# EUROPÄISCHE PATENTSCHRIFT

(11) **EP 1 165 028 B1**
(45) Veröffentlichungstag und Bekanntmachung des Hinweises auf die Patenterteilung: **18.08.2004**
(21) Anmeldenummer: 00909298.2
(22) Anmeldetag: 03.03.2000
(51) Int. Cl.: A61K 7/16

(54) **VERWENDUNG VON OBERFLÄCHENAKTIVEN GEMISCHEN**
USE OF SURFACTANT MIXTURES
UTILISATION DE MELANGES TENSIOACTIFS

(30) Priorität: 12.03.1999 DE 19911055
(43) Veröffentlichungstag der Anmeldung: 02.01.2002
(73) Patentinhaber: Biotec Pharmacon ASA, 9008 Tromsö (NO)
(72) Erfinder: GRIESBACH, Ute, D-40597 Düsseldorf (DE); WACHTER, Rolf, D-40595 Düsseldorf (DE); FABRY, Bernd, D-41352 Korschenbroich (DE); ENGSTAD, Rolf, E., N-9008 Tromso (NO)
(74) Vertreter: Melzer, Wolfgang, Dipl.-Ing.
(86) Internationale Anmeldenummer: PCT/EP2000/001828
(87) Internationale Veröffentlichungsnummer: WO 2000/054739

(56) Entgegenhaltungen:
- WO-A-95/23582
- WO-A-95/30022
- WO-A-95/30403
- WO-A-99/32073
- GB-A- 2 176 795
- US-A- 3 931 398
- PATENT ABSTRACTS OF JAPAN vol. 1999, no. 01, 29. Januar 1999 (1999-01-29) & JP 10 287536 A (TAKEDA CHEM IND LTD), 27. Oktober 1998 (1998-10-27)

## Beschreibung

### Gebiet der Erfindung

Die Erfindung befindet sich auf dem Gebiet der Mund- und Zahnhygiene und betrifft die Verwendung von Zubereitungen, enthaltend anionische und/oder nichtionische Tenside und ausgewählte Glucane zur Herstellung von Mund- und Zahnpflegemitteln sowie Zahnpasten einer definierten Zusammensetzung.

### Stand der Technik

Unter dem Begriff Mund- und Zahnpflegemittel versteht der Fachmann zum einen flüssige Zube-reitungen, die als Mundwasser den Mund- und Rachenraum desinfizieren, zum anderen werden damit pastöse oder seit einiger Zeit auch gelförmige Zahnreinigungsmittel verstanden. Diesen Zubereitungen ist die Anforderung durch den Verbraucher gemein, besonders schleimhautverträglich zu sein, um insbesondere bei kleinerer Verletzungen im Mund- oder Rachenraum nicht zu Irritationen Anlaß zu geben. Im Fall der Zahnplegemittel kommt hinzu, daß die Zubereitungen schaumstark, geschmacksneutral und reinigungsaktiv sein müssen.

In diesem Zusammenhang sei beispielsweise auf die Deutsche Patentanmeldung **DE-A1 4406748** (Henkel) verwiesen, aus der Mund- und Zahnpflegemittel bekannt sind, deren Tensidkomponente hauptsächlich von anionischen Tensiden vom Typ der Monoglyceridsulfate und nichtionischen Tensiden vom Typ der Alkylglucoside gebildet wird. Von Nachteil ist jedoch, daß diese Mittel des Stands der Technik im Hinblick auf Mundschleimhautverträglichkeit, Schaumstabilität und Reinigungsleistung nicht völlig befriedigend sind. Ein besonderes Problem besteht femer darin, Abrasivstoffe in Zahnpasten und insbesondere Zahngelen so zu dispergieren, daß auch bei Temperaturlagerung weder eine Agglomeration noch eine Separation stattfindet.

In diesem Zusammenhang sei auf die internationale Patentanmeldung **WO 96/34608** (Colgate) hingewiesen, aus der die Verwendung von β-Glucanen gegen Xerostomia bekannt ist. Gegenstand der **US 3,931,398** (Colgate) ist die subkutane Verabreichung von Glucanen in der Nähe der Mundhöhle zur Bekämpfung von Karies. In der Deutschen Patentanmeldung **DE-A1 3621303** (FMC) werden Gele auf Basis von β-1,3-Glucanen vorgeschlagen, die beispielsweise aus Agrobakterium gewonnen werden und in Zahnpasten eingesetzt werden können. Schließlich sind aus der **US 4,340,673** (Merck) spezielle Glucane mit Molekulargewichten oberhalb von 500.000 bekannt, welche zur Bekämpfung von Plaque eingesetzt werden.

Die Aufgabe der Erfindung hat daher darin bestanden, den geschilderten Nachteilen des Stands der Technik abzuhelfen und insbesondere Mund- und Zahnpflegemittel, speziell Zahnpasten, zur Verfü-gung zu stellen, die sich gleichzeitig durch eine optimierte Mundschleimhautverträglichkeit, durch eine verbesserte Schaum- und Reinigungsleistung, immunstimulierende und antimikrobielle Eigenschaften sowie insbesondere eine stabile Verteilung der Abrasivkörper auszeichnen.

### Beschreibung der Erfindung

Gegenstand der Erfindung ist die Verwendung von oberflächenaktiven Gemischen, enthaltend
(a) anionische und/oder nichtionische Tenside und
(b) wasserlösliche β-(1,3)-Glucane, die im wesentlichen frei von β-(1,6)-Verknüpfungen sind,
zur Herstellung von Mund- und Zahnpflegemitteln, insbesondere von Zahnpasten.

Überraschenderweise wurde gefunden, daß schon der Zusatz geringer Mengen von wasserlöslichen β-(1,3)-Glucanen, die substantiell frei von unerwünschten (1,6)-Verknüpfungen sind, zu bekannten Mundund Zahnpflegemitteln mit einem Gehalt an üblichen anionischen und/oder nichtionischen Tensiden nicht nur deren Mundschleimhautverträglichkeit verbessert, sondern auch die Plaqueentfemung unterstützt, den Schaum stabilisiert und insbesondere eine homogene und lagerstabile Verteilung von Abrasivstoffen bewirkt. Die Erfindung schließt die Erkenntnis ein, daß die Zubereitungen des weiteren antimikrobiell wirksam sind und das Immunsystem anregen. Unter Mittel sollen im Zusammenhang mit der Erfindung daher nicht ausschließlich Zahnpasten und Zahngele, sondern auch wäßrige oder alkoholbasierte Mundwässer sowie Kaugummis verstanden werden.

### Anionische und/oder nichtionische Tenside

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)-sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucaronsäurederivate, fettsäure-N-alkylglucamide, Proteinhydrolysate (insbesondere pflanzliche Produkte auf Weizenbasis), Polyolfettsäureester, Zuckerester, Sorbitanester, Palysorbate und Aminoxide. Sofern die nichtionischen Tenside Polyglycofetherketten enthalten, können diese eine konventionelle, vorzugsweise jedoch eine eingeengte Homologenverteilung aufweisen. Aus anwendungstechnischen Gründen - insbesondere Mundschleimhautverträglichkeit und Schaumvermögen - ist jedoch der Einsatz von Alkylsulfaten, Alkylethersulfaten, Monoglycerid-(ether)sulfaten, Olefinsulfonaten und Alkyl- und/oder Alkenyloligoglykosiden sowie deren Gemischen bevorzugt, die als wäßrige Pasten, vorzugsweise jedoch als wasserfreie Pulver oder Granulate, die beispielsweise im Flash-Dryer oder nach dem SKET-Verfahren erhalten werden, eingesetzt werden können.

### Alkylsulfate und Alkylethersulfate

Alkylsulfate und Alkylethersulfate, die als Komponente (a) in Frage kommen, stellen bekannte anionische Tenside dar, die großtechnisch durch Sulfatierung von primären Alkoholen - vorzugsweise Fettalkoholen oder Oxoalkaholen - oder deren Ethylenoxidanlagerungsprodukten sowie nachfolgende Neutralisation der resultierenden Schwefelsäurehalbester mit Basen hergestellt werden. Vorzugsweise folgen sie der Formel (I),

**R**^{**1**}**O(CH**_{**2**}**CH**_{**2**}**O)**_{**n**}**SO**_{**3**}**X (I)**

in der R¹ für einen linearen oder verzweigten Alkylrest mit 6 bis 22 Kohlenstoffatomen, n für 0 oder Zahlen von 1 bis 10 und X für ein Alkali- oder Erdalkalimetall, Ammonium, Alkylammonium, Alkanolammonium oder Glucammonium steht. Typische Beispiele sind die Sulfatierungsprodukte von Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linofenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen, in Form ihrer Natriumsalze. Weitere Beispiele stellen die Sulfatierungsprodukte der Addukte von 1 bis 10 Mol Ethylenoxid an die genannten Alkohole ebenfalls in Form ihrer Natriumsalze dar. Besonders bevorzugt ist der Einsatz von Natriumlaurylsulfat.

### Monoglycerid(ether)sulfate

Monoglyceridsulfate und Monoglyceridethersulfate, die ebenfalls als Komponente (a) in Frage kommen, stellen gleichfalls bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **EP-B1 0561825, EP·B1 0561999** (Henkel)]. Die im Sinne der Erfindung einzusetzenden Monoglycerid-(ether)sulfate folgen vorzugsweise der Formel (**II**), 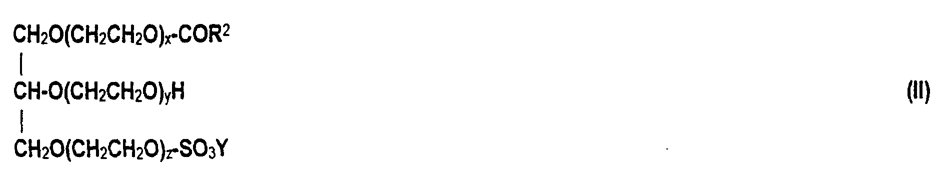 in der R²CO für einen linearen oder verzweigten Acylrest mit 6 bis 22 Kohlenstoffatomen, x, y und z in Summe für 0 oder für Zahlen von 1 bis 30, vorzugsweise 2 bis 10, und Y für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (**II**) eingesetzt, in der R¹CO für einen linearen Acylrest mit 8 bis 18 Kohlenstoffatomen steht.

### Alkyl- und/oder Alkenyloligoglykoside

Alkyl- und Alkenyloligoglykoside, die ebenfalls als Komponente (a) eingesetzt werden können, stellen bekannte nichtionische Tenside dar, die der Formel (**III**) folgen,

**R**^{**3**}**O-[G]**_{**p**} **(III)**

in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlä-gigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993)**, B.Salka in Cosm.Toil. 108, 89 (1993) sowie J.Kahre et al. in **SÖFW.Journal Heft 8, 598 (1995)** ver-wiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyl-oligoglykoside sind somit Alkyl- und/oder Alkenyloligoglucoside. Die Indexzahl p in der allgemeinen Formel (11) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Alkyl--und/oder Alkenyloligoglykoside mit einem mittleren Ofigomeri-sierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R³ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R³ kann sich ferner auch von primären Alkoholen mit 12 bis 22, vorzugsweise 12 bis 14 Kohlenstoffatomen ableiten. Typische Beispiele sind Laurytalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. **Bevorzugt** sind Alkyloligoglucoside auf Basis von gehärtetem C_{12/14}-Kokosalkohol mit einem DP von 1 bis 3.

### Wasserlösliche β-(1,3)-Glucane

Unter der Bezeichnung Glucane werden Homopolysaccharide auf Basis der Glucose verstanden. Je nach sterischer Verknüpfung unterscheidet man zwischen β-(1,3)-, β-(1,4)- und β-(1,6)-Glucanen. β-(1,3)-Glucane weisen meist eine helicale Struktur auf, während Glucane mit einer 1,4-Verknüpfung im allgemeinen eine lineare Struktur besitzen. Die β-Glucane, die im Sinne der Erfindung als Komponente (b) in Betracht kommen, besitzen eine (1,3)-Struktur, d.h. sie sind weitgehend frei von unerwünschten (1,6)-Verknüpfungen. Vorzugsweise werden solche β-(1,3)-Glucane eingesetzt, deren Seitenketten ausschließlich (1,3)-Verknüpfungen aufweisen. Insbesondere enthalten die Mittel Glucane, die auf Basis von Hefen der Familie *Saccharomyces,* speziell *Saccharomyces cerevisiae* erhalten werden. Glucane dieser Art sind in technischem Maße nach den Verfahren des Stands der Technik zugänglich. So beschreibt die Internationale Patentanmeldung **WO 95/30022** (Biotec-Mackzymal) ein Verfahren zur Herstellung solcher Stoffe, bei dem man Glucane mit β-(1,3)- und β-(1,6)-Verknüpfungen in solcher Weise mit β-(1,6)-Glucanasen in Kontakt bringt, daß praktisch alle β-(1,6)-Verknüpfungen gelöst werden. Vorzugsweise werden zur Herstellung der Glucane Glucanasen auf Basis von *Trichodermia harzianum* eingesetzt. Soweit es die Herstellung und Zugänglichkeit der in den erfindungsgemäßen Mitteln enthaltenen Glucane angeht, wird in vollem Umfang auf die Offenbarung der oben genannten Schrift Bezug genommen. Vorzugsweise liegt das Gewichtsverhältnis von Tensiden und Glucanen in den Gemischen im Bereich von 100 : 1 bis 10 : 1 und vorzugsweise 90 : 1 bis 50 : 1.

### Chitosane und Chitosanderivate

In einer bevorzugten Ausführungsform der Erfindung können die Tenside und Glucane zusammen mit Chitosanen und/oder Chitosanderivaten (Komponente c) eingesetzt werden. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten: 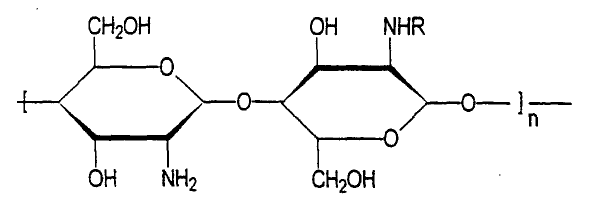 Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **UIImann's Encyclopedia of Industrial Chemistry, 5th Ed., Vol. A6, Weinheim, Verlag Chemie, 1986, S. 231-332).** Übersichten zu diesem Thema sind auch beispielsweise von B.Gesslein et al. in **HAPPI 27, 57 (1990)**, O.Skaugrud in **Drug Cosm.Ind. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 2701266** bekannt: Vorzugsweise werden solche Typen eingesetzt, wie sie in den deutschen Patentanmeldungen **DE-A1 4442987** und **DE-A1 19537001** (Henkel) offenbart werden, und die ein durchschnittliches Molekulargewicht von 10.000 bis 2.500.000, vorzugsweise 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden. Diese zeichnen sich durch eine besonders hohe Verträglichkeit mit anderen Tensiden aus.

### Hilfs und Zusatzstoffe

Die unter der erfindungsgemäßen Verwendung der oberflächenaktiven Gemische erhältlichen Zubereitungen können als **Schleif- und Poliermittel** Kreide, Dicalciumphosphat, unlösliches Natriummetaphosphat, Aluminiumsilicat, Schichtsilicate, Hydrotalcite, Calciumpyrophosphat, feinteilige Kunstharze, Kieselsäuren, Aluminiumoxid, Aluminiumoxidtrihydrat, Talkum, Zeolithe, Magnesiumaluminiumsilicat (Veegum®), Calciumsulfat, Magnesiumcarbonat und/oder Magnesiumoxid enthalten. Daneben kommen als weitere Hilfsund Zusatzstoffe schließlich **Aromakomponenten** in Frage, beispielsweise Pfefferminzöl, Krauseminzöl, Anisöl, Stemanisöl, Kümmelöl, Eukalyptusöl, Fenchel, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle, wie z.B. Menthol, Carvon, Anethol, Cineol, Eugenol, Zimtaldehyd, Cargophyllen, Geraniol, Citronellol, Linalool, Salven, Thymol, Terpinan, Terpinol, Methylchavicol und Methylsalicylat. Weitere geeignete Aromen sind z.B. Methylacetat, Vanillin, lonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam. Ferner kommen für den Einsatz insbesondere in Zahnpasten als Hilfs- und Zusatzstoffe **Feuchthaltemittel** wie z.B. Sorbit oder Glycerin, Konsistenzregler, desodorierende Wirkstoffe, Wirkstoffe gegen Mund- und Zahnerkrankungen, wasserlösliche Fluorverbindungen wie z.B. Natriumfluorid oder Natriummonofluorphosphat in Betracht. Der Anteil der Hilfs- und Zusatzstoffe ist an sich unkritisch und richtet sich nach der Art des schließlich zu konfektionierenden Mittels. Üblicherweise wird der Anteil 5 bis 98 und vorzugsweise 80 bis 90 Gew.-% - bezogen auf die Mittel - betragen. Typische Zahnpasten, die einen weiteren Gegenstand der vorliegenden Erfindung darstellen, weisen in der Regel folgende Zusammensetzung auf:
(a) 1 bis 10, vorzugsweise 2 bis 8 Gew.-% anionische und/oder nichtionische Tenside,
(b) 0,1 bis 2, vorzugsweise 0,5 bis 1 Gew.-% wasserlösliche β-(1,3)-Glucane, die im wesentlichen frei von β-(1,6)-Verknüpfungen sind,
(c) 0 bis 2, vorzugsweise 0,1 bis 1 Gew.-% Chitosane bzw. Chitosanderivate,
(d) 1 bis 25, vorzugsweise 10 bis 20 Gew.-% Schleif- und Poliermittel,
(e) 0 bis 65, vorzugsweise 10 bis 30 Gew.-% Feuchthaltemittel,
(f) 0 bis 3, vorzugsweise 1 bis 2 Gew.-% Aromastoffe und
(g) 0 bis 5, vorzugsweise 1 bis 3 Gew.-% weitere Hilfsstoffe,
mit der Maßgabe, daß sich die Einsatzmengen mit Wasser zu 100 Gew.-% ergänzen.

### Beispiele

Die Tensidgemische wurden in eine Standard-Zahnpastenrezeptur eingesetzt. Das Schaumvermögen wurde gemäß der Reibschaummethode in einem EHMEDA-Reibschaumgerät bestimmt **[Fette, Seifen, Anstrichmitt. 66**, **955 (1964)].** Hierzu wurden 20 g Zahnpaste in 180 g Wasser dispergiert und im Schaumzylinder auf 45°C erwärmt. Dort wurde durch 60 s Reibung mit einer vertikal rotierenden Perlonbürste bei 2600 UpM an einem zylindrisch geformten Metalldrahtgitter Schaum erzeugt. In Tabelle 1 ist das Schaumvolumen nach 0,5 min und 5 min nach Beendigung der Schaumerzeugung sowie das nach 5 min aus dem Schaum abgeschiedene Drainagewasser aufgeführt. Die Beurteilung der Stabilität erfolgte subjektiv nach Lagerung über 4 Wochen bei 40°C; dabei bedeutet (+) stabil, homogene Verteilung der Abrasivstoffe; (-) Agglomeration und (-) Sedimentation. Die Geschmacksbeurteilung erfolgte nach dem Zähneputzen durch 5 unabhängige Testpersonen nach folgenden Kriterien: (++) = Aroma vorherrschend, kein Beigeschmack; (+) = leichter Beigeschmack; (-) = intensiver Beigeschmack. Die Beispiele 1 bis 4 in Tabelle 1 sind erfindungsgemäß, die Beispiele V1 bis V3 dienen zum Vergleich.

**Tabelle 1**

| **Beurteilung von Zahnpasten (Mengenangaben als Gew.%)** | | | | | | | |
|---|---|---|---|---|---|---|---|
| **Zusammensetzung / Performance** | **1** | **2** | **3** | **4** | **V1** | **V2** | **V3** |
| Sodium Lauryl Sulfate | 2,0 | - | 2,0 | - | 2,0 | - | 2,0 |
| Sodium Glyceryl Cocoate Sulfate | - | 2,0 | - | 2,0 | - | 2,0 | - |
| Coco Glucosides | - | - | 0,5 | 1,0 | - | - | - |
| Betaglucane* | 0,1 | 0,1 | 0,1 | 0,1 | - | - | - |
| Chitosan** | - | - | 0,1 | 0,1 | - | - | 0,1 |
| Silica Gel | 22,0 | 22,0 | 22,0 | 22,0 | 22,0 | 22,0 | 22,0 |
| Sodium Carboxymethyl Cellulose | 1,2 | 1,2 | 1,2 | 1,2 | 1,2 | 1,2 | 1,2 |
| Saccharin, Sodium Salt | 0,1 | 0,1 | 0,1 | 0,1 | 0,1 | 0,1 | 0,1 |
| Sodium Benzoate | 0,1 | 0,1 | 0,1 | 0,1 | 0,1 | 0,1 | 0,1 |
| Sodium Fluoride | 0,2 | 0,2 | 0,2 | 0,2 | 0,2 | 0,2 | 0,2 |
| Sorbitol (70 Gew.-%ig) | 15,0 | 15,0 | 15,0 | 15,0 | 15,0 | 15,0 | 15,0 |
| Glycerol (86 Gew.-%ig) | 25,0 | 25,0 | 25,0 | 25,0 | 25,0 | 25,0 | 25,0 |
| Flavour | 3,0 | 3,0 | 3,0 | 3,0 | 3,0 | 3,0 | 3,0 |
| Wasser | ad 100 | | | | | | |
| ***Reibeschaum [ml]*** | | | | | | | |
| - *nach 0,5 min* | 780 | 810 | 820 | 830 | 740 | 760 | 780 |
| - *nach* 5 *min* | 600 | 610 | 610 | 640 | 530 | 530 | 520 |
| - *Drainagewasser* | 60 | 55 | 55 | 60 | 70 | 65 | 75 |
| *Stabilität* | + | + | + | + | - | - | - |
| *Geschmackliche Beurteilung* | + | + | + | ++ | - | + | + |

| | | | | | | | |
|---|---|---|---|---|---|---|---|
| *) Highcareen® GS, | | | | | | | |
| **) Hydagen® CMF (beides Henkel KGaA, Düsseldorf/FRG) | | | | | | | |

## Patentansprüche

1. Verwendung von oberflächenaktiven Gemischen, enthaltend
(a) anionische und/oder nichtionische Tenside und
(b) wasserlösliche β-(1,3)-Glucane, die im wesentlichen frei von β-(1,6)-Verknüpfungen sind,
zur Herstellung von Mund- und Zahnpflegemitteln.

2. Verwendung nach Anspruch 1, **dadurch gekennzeichnet, daß** man als Komponente (a) anionische Tenside einsetzt, die ausgewählt sind aus der Gruppe, die von Alkylsulfaten, Alkylethersulfaten, Monoglycerid(ether)sulfaten und Olefinsulfonaten gebildet wird.

3. Verwendung nach den Ansprüchen 1 und/oder 2, **dadurch gekennzeichnet, daß** man als Komponente (a) nichtionische Tenside vom Typ der Alkyl- und/oder Alkenyloligoglykoside einsetzt.

4. Verwendung nach mindestens einem der Ansprüche 1 bis 3, **dadurch gekennzeichnet, daß** man als Komponente (b) wasserlösliche β-(1,3)-Glucane einsetzt, die auf Basis von Hefen der Familie *Saccharomyces* erhalten werden.

5. Verwendung nach Anspruch 4, **dadurch gekennzeichnet, daß** man Glucane einsetzt, die erhalten werden, indem man Glucane mit β-(1,3)- und β-(1,6)-Verknüpfungen in solcher Weise mit β-(1,6)-Glucanasen in Kontakt bringt, daß praktisch alle β-(1,6)-Verknüpfungen gelöst werden.

6. Verwendung nach Anspruch 5, **dadurch gekennzeichnet, daß** man Glucane einsetzt, die zuvor mit Glucanasen auf Basis von *Trichodermia harzianum* behandelt worden sind.

7. Verwendung nach mindestens einem der Ansprüche 1 bis 6, **dadurch gekennzeichnet, daß** man die Tenside und die Glucane im Gewichtsverhältnis 100 : 1 bis 10 : 1 einsetzt.

8. Verwendung nach mindestens einem der Ansprüche 1 bis 7, **dadurch gekennzeichnet, daß** man als weitere Komponente (c) Chitosane und/oder Chitosanderivate einsetzt.

9. Verwendung nach mindestens einem der Ansprüche 1 bis 8, **dadurch gekennzeichnet, daß** man Zahnpasten, Zahngele, Mundwässer oder Kaugummis herstellt.

10. Zahnpasten, enthaltend
(a) 1 bis 10 Gew.-% anionische und/oder nichtionische Tenside,
(b) 0,1 bis 2 Gew.-% wasserlösliche β-(1,3)-Glucane, die im wesentlichen frei von β-(1,6)-Verknüpfungen sind,
(c) 0 bis 2 Gew.-% Chitosane bzw. Chitosanderivate,
(d) 1 bis 25 Gew.-% Schleif- und Poliermittel,
(e) 0 bis 65 Gew.-% Feuchthaltemittel,
(f) 0 bis 3 Gew.-% Aromastoffe und
(g) 0 bis 5 Gew.-% weitere Hilfsstoffe,
mit der Maßgabe, daß sich die Einsatzmengen mit Wasser zu 100 Gew.-% ergänzen.

## Claims

1. Use of surface-active mixtures comprising
(a) anionic and/or nonionic surfactants and
(b) water-soluble β-(1,3)-glucans which are essentially free from β- (1, 6) linkages,
for the preparation of oral and dental care compositions.

2. Use according to Claim 1, **characterized in that** anionic surfactants chosen from the group formed by alkyl sulphates, alkyl ether sulphates, monoglyceride (ether) sulphates and olefinsulphonates are used as component (a).

3. Use according to Claims 1 and/or 2, **characterized in that** nonionic surfactants of the alkyl and/or alkenyl oligoglycoside type are used as component (a).

4. Use according to at least one of Claims 1 to 3, **characterized in that** water-soluble β-(1,3)-glucans which have been obtained on the basis of yeasts of the *Saccharomyces* family are used as component (b).

5. Use according to Claim 4, **characterized in that** glucans which are obtained by bringing glucans with β-(1,3) and β-(1,6) linkages into contact with β-(1,6)-glucanases in such a way that virtually all of the β-(1,6) linkages are broken are used.

6. Use according to Claim 5, **characterized in that** glucans which have been treated beforehand with glucanases based on *Trichodermia harzianum* are used.

7. Use according to at least one of Claims 1 to 6, **characterized in that** the surfactants and the glucans are used in the weight ratio 100:1 to 10:1.

8. Use according to at least one of Claims 1 to 7, **characterized in that** chitosans and/or chitosan derivatives are used as further component (c).

9. Use according to at least one of Claims 1 to 8, **characterized in that** toothpastes, tooth gels, mouth washes or chewing gums are prepared.

10. Toothpastes comprising
(a) 1 to 10% by weight of anionic and/or nonionic surfactants,
(b) 0.1 to 2% by weight of water-soluble β-(1,3) glucans which are essentially free from β-(1,6) linkages,
(c) 0 to 2% by weight of chitosans and/or chitosan derivatives,
(d) 1 to 25% by weight of abrasive and polishing agents,
(e) 0 to 65% by weight of humectants,
(f) 0 to 3% by weight of aroma substances and
(g) 0 to 5% by weight of further auxiliaries,
with the proviso that the use amounts are made up to 100% by weight with water.

## Revendications

1. Utilisation de mélanges tensioactifs contenant :
(a) des tensioactifs anioniques et/ou non ioniques et
(b) des β-(1,3)-glucanes hydrosolubles qui sont sensiblement dépourvus de liaisons β-(1,6),
pour la production d'agents de soins buccaux et dentaires.

2. Utilisation selon la revendication 1, **caractérisée en ce que** l'on utilise comme composant (a) des tensioactifs anioniques qui sont choisis dans le groupe qui est formé par les alkylsulfates, les alkyléthersulfates, les monoglycéride(éther)sulfates et les oléfine-sulfonates.

3. Utilisation selon les revendications 1 et/ou 2, **caractérisée en ce que** l'on utilise comme composant (a) des tensioactifs non ioniques du type des alkyl- et/ou alcényloligoglycosides.

4. Utilisation selon au moins l"une des revendications 1 à 3, **caractérisée en ce que** l'on utilise comme composant (b) des β-(1,3)-glucanes hydrosolubles qui sont obtenus à base de levures de la famille *Saccaromyces*.

5. Utilisation selon la revendication 4, **caractérisée en ce que** l'on utilise des glucanes qui sont obtenus en mettant en contact des glucanes ayant des liaisons β-(1,3) et β-(1,6) avec des β-(1,6)-glucanases de telle manière que pratiquement toutes les liaisons β-(1,6) sont clivées.

6. Utilisation selon la revendication 5, **caractérisée en ce que** l'on utilise des glucanes qui ont été traités au préalable avec des glucanases à base de *Trichodermia harzianum.*

7. Utilisation selon au moins l'une des revendications 1 à 6, **caractérisée en ce que** l'on utilise les tensioactifs et les glucanes dans le rapport massique 100:1 à 10:1.

8. Utilisation selon au moins l'une des revendications 1 à 7, **caractérisée en ce que** l'on utilise comme composant supplémentaire (c) des chitosanes et/ou des dérivés de chitosanes.

9. Utilisation selon au moins l'une des revendications 1 à 8, **caractérisée en ce que** l'on produit des pâtes dentifrices, des gels dentifrices, des bains de bouche ou des gommes à mâcher.

10. Pâtes dentifrices contenant :
(a) 1 à 10 % en masse de tensioactifs anioniques et/ou non ioniques,
(b) 0,1 à 2 % en masse de β-(1,3)-glucanes hydrosolubles qui sont sensiblement dépourvus de liaisons β-(1,6),
(c) 0 à 2 % en masse de chitosanes ou de dérivés de chitosanes,
(d) 1 à 25 % d'abrasifs et d'agents de polissage,
(e) 0 à 65 % en masse d'agents de maintien de l'humidité,
(f) 0 à 3 % en masse de substances aromatisantes et
(g) 0 à 5 % en masse d'autres adjuvants,
avec la condition que les quantités utilisées totalisent 100 % en masse avec l'eau.
